# EUROPEAN PATENT APPLICATION

(11) **EP 4 840 415 A1**
(43) Date of publication of application: **18.11.2026**
(21) Application number: 25176608.5
(22) Date of filing: 15.05.2025
(51) Int. Cl.: A61B 18/18, A61B 18/00, A61B 18/20

(54) **A PERSONAL CARE DEVICE FOR APPLICATION AGAINST THE SKIN**

(71) Applicant: Koninklijke Philips N.V., 5656 AE Eindhoven (NL)
(72) Inventor: ZHANG, Jun, 5656 AG Eindhoven (NL); LIN, Xiaoyu, 5656 AG Eindhoven (NL); TAN, Liangwei, 5656AG Eindhoven (NL); DAI, Zifei, 5656 AG Eindhoven (NL)
(74) Representative: Philips Intellectual Property & Standards

(57) **Abstract**

A personal care device for light-based treatment of the skin has a power supply circuit for processing a mains supply. A first switch mode power supply comprises a boost converter for boosting a rectified mains signal to create a boosted DC voltage signal and a second switch mode power supply comprises a buck converter for reducing the rectified mains signal to create a reduced DC voltage signal. A capacitor is used to store the boosted DC voltage signal and a light source is powered by charge stored on the capacitor. A control circuit is powered by the reduced DC voltage signal for controlling the timing of operation of the light source. This separation of switch mode power supplies enables improved thermal management.

## Description

### FIELD OF THE INVENTION

This invention relates to light based skin treatment or hair removal devices.

### BACKGROUND OF THE INVENTION

Light-based epilators are well-known. People use hair epilators or depilators to remove unwanted hair. Typical target areas for women are the face, armpit, arm, leg, bikini line, and body. Men use light-based epilators also on the chest and back. Unwanted hair is removed by targeting the hair follicles. These devices are designed to provide a long-lasting reduction in hair growth.

Light-based hair removal devices typically use laser light or are based on the filtered output of a flashlamp, known as Intense Pulsed Light or IPL.

Lasers emit a focused, single-wavelength beam of light, while IPL devices produce a broad spectrum of light. The light produced by these sources is selectively absorbed by the pigment (melanin) in the hair. The primary target of light hair epilation device is the melanin present in the hair shaft. When the device emits a pulse of light, the melanin in the hair absorbs this light energy, which is then converted into heat. The heat generated in the hair shaft during this process damages the hair follicle. It can effectively disable the follicle's ability to grow hair, resulting in long-term hair reduction. This process is called selective photothermolysis.

A light-based hair epilation device typically comprises several key components to generate and deliver the laser or intense pulsed light for various applications, including hair removal and skin rejuvenation. These components may vary in design and functionality based on the specific make and model of the device, but the fundamental elements include:
a light source, such as a laser lamp or xenon lamp;
a reflective surface to direct the light energy towards the treatment area. It maximizes the efficiency of light delivery;
filters to modify the light spectrum emitted. Filters can target specific wavelengths of light to optimize the treatment for various applications, such as hair removal, skin pigmentation, or vascular treatments; and
a handpiece or applicator for making direct contact with the skin. It usually contains the light source and the optical components needed to focus and deliver the light energy effectively.

Modern devices also incorporate user-friendly control panels or touchscreens that allow users to adjust settings such as intensity levels, pulse duration, and other parameters. These settings are selected based on the individual's skin type and treatment area.

To enhance user comfort and protect the epidermis from excessive heat, many devices incorporate a cooling mechanism. This may include contact cooling (cooling the applicator tip) or integrated cooling systems to maintain skin temperature during the procedure.

The device is powered by an energy source to generate and deliver the laser or intense pulsed light. The device is typically powered from an electrical outlet.

Safety features are typically also provided such as protective eyewear for the user and the patient to shield the eyes from the intense light. Some devices also include built-in safety sensors to prevent accidental flashes and control the delivery of light. Additional sensors may be used to ensure skin contact and measure the skin's melanin levels to adapt treatment parameters for optimal results and safety. Some devices offer pre-set treatment programs for specific applications, making it easier for users to select the correct settings for hair removal, skin rejuvenation, or other treatments.

Light hair epilation devices are a popular choice for those seeking long-term hair reduction because of their effectiveness and ease of use. However, it is crucial to use them correctly and their performance depends on the efficiency of hair removal.

Normally, an at-home light hair epilation device is designed with low power circuitry. It typically involves spot-by-spot treatment, by moving the handpiece across the skin in a controlled and overlapping manner. It ensures that all the hair follicles in the target treatment area receive the necessary light energy to be effective.

For example, the power supply system for a light-based treatment device typically comprises a switch mode power supply which converts a mains input to a 24V DC safety voltage which is provided to the device. Another circuit board boosts the DC safety voltage up to a high voltage level such as 300V DC or 400V DC to supply a high voltage capacitor to drive the light source. This design requires a large input current from the switch mode power supply and requires a significant space for the 24V DC to 300V or 400V boost board. This also has poor power transfer efficiency and hence has limited power to supply a more intense light pulse.

The DC safety input voltage may also be reduced by a buck converter to a control voltage (e.g. 12V) for supplying a control circuit.

There is a need for an improved power supply system.

### SUMMARY OF THE INVENTION

The invention is defined by the claims.

According to examples in accordance with an aspect of the invention, there is provided a personal care device for application against the skin, comprising:
an input for receiving a mains supply;
a rectifier arrangement for generating a rectified mains signal;
a first switch mode power supply comprising a boost converter for boosting the rectified mains signal to create a boosted DC voltage signal;
a second switch mode power supply comprising a buck converter for reducing the rectified mains signal to create a reduced DC voltage signal;
a capacitor for storing the boosted DC voltage signal;
a light source for generating a light output, powered by charge stored on the capacitor; and
a control circuit powered by the reduced DC voltage signal for controlling the timing of operation of the light source.

This device has a power supply system which combines a boost converter and a buck converter. The mains AC signal, such as 220V AC. is rectified for example to 310V DC. The boost converter directly boosts up to a boosted voltage such as 400V DC to drive the light source. The buck converter steps down from the mains AC signal to drive the control circuit.

In this way, there are two independent power supplies, each supplied by the original (or rectified) mains supply signal. One directly modulates the mains voltage, such as 220V AC, to the boosted DC voltage, such as 400V DC. Most of the heat generation in the circuitry is caused by driving the light source, hence by this first power supply. By providing a power supply dedicated just to the driving of the light source, the heat dissipation is more efficient and it can be cooled separately from the other components of the device. In this way, the power supply to the light source can be larger. The other power supply is smaller for the conversion from the mains supply, such as 220V AC to the reduced DC voltage, such as 12V DC.

The rectifier arrangement for example comprises a first rectifier which is part of the first switch mode power supply and a second rectifier which is part of the second switch mode power supply.

Thus, each switch mode power supply receives the raw mains signal, and performs all signal processing to reach the desired output. As an alternative, a single rectifier could be used that supplies the rectified mains signal to each of the switch mode power supplies.

The boosted DC voltage signal is for example at least 390V DC. The boosted DC voltage is for example 400V.

The light output may be generated as pulses with a maximum operational frequency of least 3 Hz. Typically, IPL treatment devices operate at around 1Hz. By running at a higher frequency, the user can move the device more smoothly over the skin. The power supply deign enables heat dissipation to be managed to enable the higher power operation of the switch mode power supply. The power output of the first switch mode power supply is for example 300W.

The device may further comprise a heat dissipation system.

The heat dissipation system for example comprises a fan that is powered by the reduced DC voltage signal. The fan for example draws ambient air from an air inlet over the light source using the fan. Ab air outlet expels the heated air away from the light source.

The heat dissipation system for example also comprises a fan driver that is powered by the reduced DC voltage signal.

The device may further comprise a voltage reducing circuit for further reducing the reduced DC voltage signal for powering one or more of a fan, LEDs, and the control circuit. The reduced DC voltage signal is for example 24V. It is used to power the control circuit and other components (which may all be considered to be low voltage components, relative to the light source), but in this case there is a voltage down-conversion before the reduced DC voltage is provided to one or more of those low voltage components. For example, the further reduced voltage may be 12V.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows the main components of an intense pulsed light device for application to the skin, for skin or hair treatment; and
Fig. 2 shows a power supply circuit used in the device of Fig. 1.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a personal care device for light-based treatment of the skin, having a power supply circuit for processing a mains supply. A first switch mode power supply comprises a boost converter for boosting a rectified mains signal to create a boosted DC voltage signal and a second switch mode power supply comprises a buck converter for reducing the rectified mains signal to create a reduced DC voltage signal. A capacitor is used to store the boosted DC voltage signal and a light source is powered by charge stored on the capacitor. A control circuit is powered by the reduced DC voltage signal for controlling the timing of operation of the light source. This separation of switch mode power supplies enables improved thermal management.

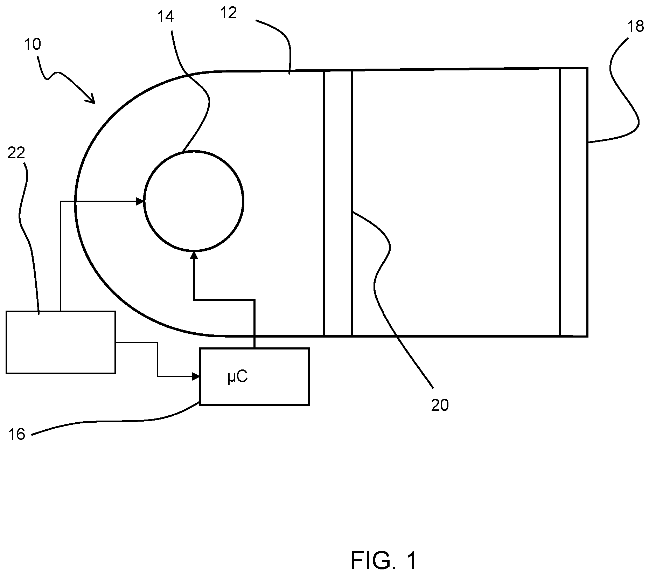

Fig. 1 shows a schematic representation of the main functional parts of a device 10 for hair removal or treatment of skin, and in particular shows an IPL device as an example. The device comprises an outer housing which contains a light source 14, such as a flashlamp 14, for generating treatment light in this example. A controller 16 is provided for controlling the light source. The light source delivers treatment light to the skin of the user (and hence to hairs of the user on the skin surface) through an output window 18. The outer housing comprises a reflector 12. An optical filter 20 is provided between the light source 14 and the output window. It divides the reflector into two parts - a lamp reflector around the lamp and a tube reflector.

The filter 20 controls the optical characteristics of the light delivered to the skin. The filter for example prevents shorter wavelengths reaching the skin, as they may be harmful. The wavelengths of light are for example tuned for absorption by melanin. A reflective filter experiences reduced heating compared to an absorbing filter.

The light source 14 for example comprises a Xenon flashlamp. The flashlamp is surrounded by reflecting surfaces, including a curved rear reflector surface 12 which defines the lamp reflector. The curved reflector shapes the light from the flashlamp into an output beam directed to the output window 18. The tube reflector part ensures that all light from the Xenon flashlamp reaches a front plate which includes the output window 18, either directly or after one or more reflections.

The light source 14 and controller 16 are powered by a power supply circuit 22. In known systems, the power supply circuit is fed by a mains AC input, such as 110V or 220V AC. The power supply circuit converts the mains input to a 24V DC safety voltage which is provided to the rest of the device. This device input voltage is reduced by a buck converter to a control voltage (e.g. 12V) for supplying the control circuit 16. Another circuit board boosts the DC safety voltage up to a high voltage level such as 300V DC to supply a high voltage capacitor to drive the light source.

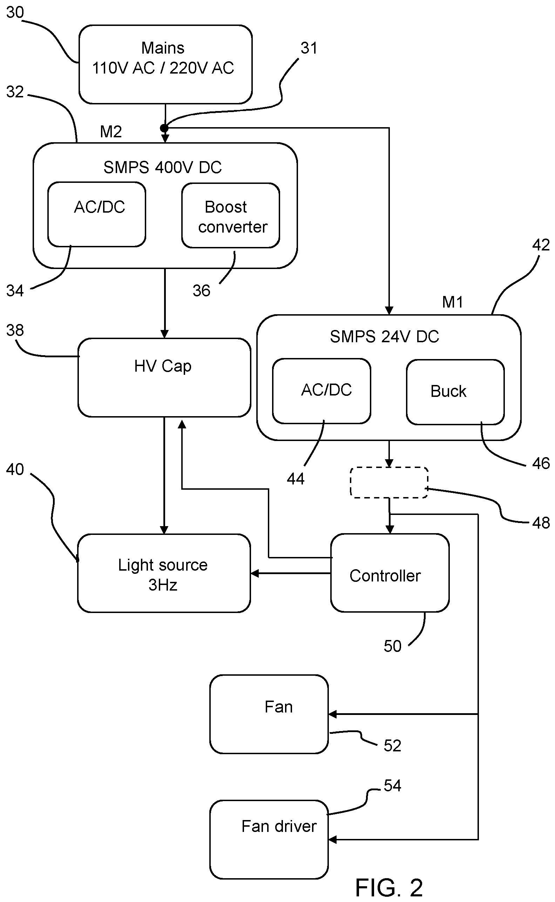

This invention relates to an improved power supply circuit, as shown in Fig. 2. Fig. 2 shows the power supply circuit together with the components powered by the circuit.

The power supply circuit has an input 31 for receiving a mains supply. The mains supply is for example a 110V AC signal or a 220V or 240V AC signal.

A first switch mode power supply 32 comprises a first rectifier 34 and a boost converter 36 for boosting the rectified mains signal (which is for example 310V AC) to create a boosted DC voltage signal. The boosted DC voltage signal is for example at least 390V, for example 400V.

A second switch mode power supply 42 comprises a second rectifier 44 and a buck converter 46 for reducing the rectified mains signal to create a reduced DC voltage signal. The reduced DC voltage signal is for example 24V.

A high voltage capacitor 38 is provided for storing the boosted DC voltage signal. The charge stored on the capacitor 38 is used to deliver charge to the light source 40 so that a flashed light output is generated.

A control circuit 50 controls the charging and discharging of the capacitor 38 and thereby controls the timing of operation of the light source 40. The control circuit 50 is powered by the reduced DC voltage signal.

This device thus has a power supply system which combines a boost converter and a buck converter as separate switch mode power converters. They for example comprise circuits each on their own respective printed circuit board. The mains AC signal, such as 220V AC, is rectified to around 310V DC. The boost converter directly boosts up to a boosted voltage such as 400V DC to drive the light source. The buck converter steps down from the rectified mains AC signal to drive the control circuit.

By providing a power supply only for charging the capacitor, the heat generation is kept to a minimum so that heat dissipation can be more efficient.

The first and second rectifiers may be considered to comprise a rectifier arrangement. The rectifier arrangement may instead have a only single rectifier that supplies both of the switch mode power supplies with a rectified AC signal, such as a 310V DC signal.

The improved heat dissipation enables the flashed light output to be generated at a higher frequency, such as 3Hz. This requires a higher power power supply such as 300W. By running at 3Hz, the device can apply light to an area three times the size of the light output window of the device in each second. This means that the device can be moved gradually across the skin at a rate of approximately 50mm per second. This enables users to smoothly slide or glide the handpiece.

As mentioned above, a heat dissipation may be used to remove heat from the light source (and optionally also its power supply). For example, a flow of air may be generated by fan 52 that is powered by the reduced DC voltage signal. The fan is controlled by fan driver 54 that is also powered by the reduced DC voltage signal.

Fig. 2 shows that the 24V "reduced DC voltage" may be further reduced by an optional voltage reducing circuit 48. The voltage down-conversion in circuit 48 for example delivers a 12V DC signal. This is more efficient than converting from the mains AC signal directly to 12V DC for example because larger power up capacitors would be required.

To ensure efficient energy use, the device is for example arranged such that light is generated only after the device has moved over the skin by a distance equivalent to the width or length of the treatment window of the device. For this purpose, an optical sensor may be used to continuously capture images of the skin surface. Image analysis then enables pattern changes to be detected, and in this way the movement of the device can be accurately tracked. When the movement distance reaches a distance equal to the width or length of the treatment window, the sensor sends a signal to the controller. The controller then activates the light source to deliver a flash, for example for hair epilation. This approach maximizes the device's efficiency and effectiveness during use.

Since the high-power power supply drives the light source, significant heat is generated. The thermal dissipation approach mentioned above effectively manages heat-related issues. It includes an air inlet, a fan module drawing in ambient-temperature air through the air inlet, an air channel directing the ambient temperature air over the light source propelled by the fan module, and an air outlet expelling the heated air from the light source.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a controller may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A personal care device for application against the skin, comprising:
an input (31) for receiving a mains supply;
a rectifier arrangement (34,44) for generating a rectified mains signal;
a first switch mode power supply (32) comprising a boost converter (36) for boosting the rectified mains signal to create a boosted DC voltage signal;
a second switch mode power supply (42) comprising a buck converter (46) for reducing the rectified mains signal to create a reduced DC voltage signal;
a capacitor (38) for storing the boosted DC voltage signal;
a light source (40) for generating a light output, powered by charge stored on the capacitor (38); and
a control circuit (50) powered by the reduced DC voltage signal for controlling the timing of operation of the light source (40).

2. The device of claim 1, wherein the rectifier arrangement comprises a first rectifier (34) which is part of the first switch mode power supply (32) and a second rectifier (44) which is part of the second switch mode power supply (42).

3. The device of claim 1, wherein the boosted DC voltage signal is at least 390V DC.

4. The device of any one of claims 1 to 3, wherein the light output is generated as pulses with a maximum operational frequency of least 3 Hz.

5. The device of any one of claims 1 to 4, further comprising a heat dissipation system.

6. The device of claim 5, wherein the heat dissipation system comprises a fan (52) that is powered by the reduced DC voltage signal.

7. The device of claim 6, wherein the heat dissipation system comprises a fan driver (54) that is powered by the reduced DC voltage signal.

8. The device of any one of claims 1 to 7, further comprising a voltage reducing circuit (48) for further reducing the reduced DC voltage signal for powering one or more of a fan, LEDs, and the control circuit.
